# EUROPÄISCHE PATENTSCHRIFT

(11) **EP 2 671 531 B1**
(45) Veröffentlichungstag und Bekanntmachung des Hinweises auf die Patenterteilung: **20.01.2016**
(21) Anmeldenummer: 13182546.5
(22) Anmeldetag: 24.08.2010
(51) Int. Cl.: A61C 3/02, A61B 17/16, A61C 1/08, A61C 1/00, A61C 8/00, A61C 1/05

(54) **Medizinisches, insbesondere dentales, Bohrwerkzeug**
Medical, in particular dental drilling tool
Outil de forage médical, notamment dentaire

(43) Veröffentlichungstag der Anmeldung: 11.12.2013
(62) Teilanmeldung aus: 10173768.2
(73) Patentinhaber: W & H Dentalwerk Bürmoos GmbH, 5111 Bürmoos (AT)
(72) Erfinder: Pruckner, Christian, 1190 Wien (AT); Jindra, Thomas, 5121 Ostermiething (AT); Watzek, Georg, 1030 Wien (AT); Unger, Ewald, 1210 Wien (AT)
(74) Vertreter: Benda, Ralf

(56) Entgegenhaltungen:
- EP-A2- 1 269 933
- US-A1- 2008 293 010
- US-A1- 2010 121 330

## Beschreibung

Die vorliegende Erfindung betrifft ein medizinisches, insbesondere dentales, Bohrwerkzeug gemäß

Anspruchs 1, zur Verwendung in eine Vorrichtung für den Schnellstopp des Bohrwerkzeugs.

Eine derartige Vorrichtung ist zum Beispiel aus der Patentanmeldung DE 10 2008 032 704 A1 bekannt. Die Vorrichtung umfasst ein Bohrwerkzeug mit zwei konzentrischen, axial gegeneinander verschiebbaren Teilen, wobei das Innenteil gegenüber dem Außenteil axial in Bohrrichtung mittels einer Feder vorgespannt ist und ohne oder bei zu geringer Kraftgegenbeaufschlagung mit seiner Spitze gegenüber der Spitze des Außenteils in Bohrrichtung etwas vorsteht. Es ist des Weiteren eine Einrichtung vorgesehen, die bei einer axialen Verschiebung des Innenteils gegenüber dem Außenteil den Antrieb des Bohrwerkzeugs unterbricht, wobei die Einrichtung mit einem elektromagnetischen Sensor verbunden ist, der die axiale Verschiebung des Innenteils zum Außenteil detektiert.

Der Sensor ist als Endlagensensor ausgebildet, der an dem rückwärtigen (den Anschluss an einen Antrieb aufweisenden) Ende des Bohrwerkzeugs angeordnet ist und eine Bewegung des verbreiterten Endes des Innenteils, das aus dem Außenteil des Bohrwerkzeugs heraussteht, detektiert. Dieser Aufbau ist jedoch von erheblichem Nachteil wenn die Vorrichtung für den Schnellstopp in einen Handstückkopf, insbesondere in einen Winkelstückkopf, integriert werden soll, da insbesondere aufgrund der beengten Platzverhältnisse im Handstückkopf kaum Raum für den Sensor und für den Stellweg des verbreiterten Endes des Innenteils vorhanden ist. Zusätzlich behindert der Endlagensensor den Anschluss des Bohrwerkzeugs an eine Fluidversorgung. Ein derartiger Bohrwerkzeug ist auch aus der Patentanmeldung US 2008/0293010 bekannt.

Beschrieben wird eine Vorrichtung für den Schnellstopp eines medizinischen, insbesondere dentalen, Bohrwerkzeugs, die aufweist: Ein Bohrwerkzeug mit einem Anschlussende zur Verbindung mit einem Antrieb, einem abrasiven Arbeitsende zum Materialabtrag und einem sich zwischen dem Anschlussende und dem Arbeitsende erstreckenden Körper mit einer sich entlang einer Längsachse des Bohrwerkzeugs erstreckenden Längsausdehnung, wobei das Bohrwerkzeug eine hohle Außenhülse aufweist, in welcher ein durch ein Federelement vorgespannter Fühler aufgenommen ist, der relativ zur Außenhülse entlang der Längsachse bewegbar ist, so dass zumindest ein Teil des Fühlers durch eine Öffnung der Außenhülse am Arbeitsende aus der Außenhülse bewegbar ist, wobei der Fühler als länglicher, sich entlang der Längsachse erstreckender Stift mit einem ersten, dem Anschlussende zugewandten Ende und einem zweiten, dem Arbeitsende zugewandten Ende ausgebildet ist, und einen elektromagnetischen Sensor zur Detektion einer Relativbewegung zwischen der Außenhülse und dem Fühler, wobei das erste, dem Anschlussende zugewandte Ende des Fühlers im Inneren der hohlen Außenhülse des Bohrwerkzeugs aufgenommen ist, der elektromagnetische Sensor entlang der Längsausdehnung des Körpers des Bohrwerkzeugs angeordnet ist und das Bohrwerkzeug einen sich entlang der Längsachse erstreckenden Leitungskanal für ein Behandlungsfluid aufweist.

Durch die Anordnung des elektromagnetischen Sensors entlang der Längsausdehnung des Körpers des Bohrwerkzeugs und des ersten, dem Anschlussende zugewandte Ende des Fühlers im Inneren der hohlen Außenhülse des Bohrwerkzeugs wird im Handstück, am rückwärtigen Ende (Anschlussende) des Bohrwerkzeugs kein zusätzlicher Patz für den Sensor benötigt. Somit besteht des Weiteren die Möglichkeit das Bohrwerkzeug an dessen Anschlussende mit einer Fluidquelle für ein Behandlungsfluid und / oder Kühlfluid zu verbinden und das Behandlungsfluid durch das Bohrwerkzeug entlang eines sich der Längsachse erstreckenden Leitungskanal zu leiten.

Als elektromagnetischer Sensor im Sinne der vorliegenden Schrift werden sowohl Sensoren verstanden, die primär oder ausschließlich auf elektrische Felder reagieren, zum Beispiel kapazitive Sensoren, als auch Sensoren, die primär oder ausschließlich auf magnetische Felder reagieren, zum Beispiel induktive Sensoren oder Magnetsensoren.

Gemäß einem Ausführungsbeispiel weist der elektromagnetische Sensor einen induktiven Sensor mit zumindest einer Spule und einem Spulenkern, zum Beispiel einem hart- oder weichmagnetischen Magnetelement, insbesondere einem Ferritkörper, auf, wobei die zumindest eine Spule und der Spulenkern relativ zueinander bewegbar sind, insbesondere durch die Relativbewegung des Fühlers zur Außenhülse. Bevorzugt ist der Spulenkern mit dem Fühler entlang der Längsachse des Bohrwerkzeugs und relativ zur zumindest einen Spule bewegbar. Unter einem induktiven Sensor wird sowohl ein Sensor verstanden, der aufgrund der im Vorstehenden genannten Relativbewegung eine Induktivitätsänderung in der Spule detektiert (wobei dieser Sensor ein weichmagnetisches Magnetelement aufweist), als auch ein Sensor, welcher aufgrund der im Vorstehenden genannten Relativbewegung eine in der Spule erzeugte Induktionsspannung detektiert (wobei dieser Sensor ein permanentmagnetisches Magnetelement aufweist).

Gemäß einem Ausführungsbeispiel weist der elektromagnetische Sensor einen Magnetsensor, insbesondere einen Hall-Sensor oder einen Reed-Sensor, und zumindest ein Magnetelement auf, wobei der Magnetsensor und das zumindest eine Magnetelement durch die Relativbewegung des Fühlers zur Außenhülse relativ zueinander bewegbar sind.

Gemäß einem Ausführungsbeispiel weist der elektromagnetische Sensor einen kapazitiven Sensor mit zumindest zwei metallischen Elektroden auf, die einen Kondensator bilden, wobei eine Elektrode durch die Relativbewegung des Fühlers zur Außenhülse relativ zu einer anderen Elektrode bewegbar ist. Bevorzugt umfasst der kapazitive Sensor zumindest zwei im Wesentlichen plattenförmige Elektroden sowie eine mit dem Fühler entlang der Längsachse des Bohrwerkzeugs und relativ zu den zwei im Wesentlichen plattenförmigen Elektroden bewegbare Messelektrode.

Die vorgenannten Ausführungsbeispiele, insbesondere die verwendeten Sensoren, haben mehrere Vorteile: Die Sensoren sind als berührungslose Sensoren ausgebildet und arbeiten im Wesentlichen verschleißfrei. Die Abmessungen der Sensoren sind gering, so dass zumindest Teile der Sensoren im Bohrwerkzeug integriert werden können und / oder am oder in der Nähe des Bohrwerkzeugs angebracht werden können, ohne die Verwendung des Bohrwerkzeugs zu stören oder zu beeinflussen. Die Sensoren oder zumindest Teile davon sind gegenüber äußeren Einflüssen, wie zum Beispiel Behandlungsfluiden, Flüssigkeiten, Dämpfen, Reinigungsmitteln, Partikeln, resistent oder zumindest Teile der Sensoren sind in einer Kapselung integrierbar, um gegenüber den äußeren Einflüssen resistent zu sein. Insbesondere können zumindest Teile der Sensoren, zum Beispiel ein Magnetelement, ein Ferritkörper oder eine Platte/Elektrode des Kondensators derart verkleinert werden, dass sie in der hohlen Außenhülse / im Inneren der hohlen Außenhülse des Bohrwerkzeugs anordenbar sind, besonders bevorzugt mit dem Fühler verbunden, und trotz ihrer Verkleinerung überraschender Weise ein ausreichend starkes, auswertbares oder verarbeitbares Sensorsignal erzeugen. Der Durchmesser oder die Breite eines mit dem Fühler verbundenen Sensorelements, zum Beispiel eines Magnetelements, eines Ferritkörpers oder einer Platte/Elektrode des Kondensators, beträgt bevorzugt weniger als 3,0 mm, besonders bevorzugt weniger als 2,5 mm.

Gemäß einem Ausführungsbeispiel weist der Leitungskanal für ein Behandlungsfluid eine Bohrung in der Außenhülse des Bohrwerkzeugs auf und / oder durchsetzt das den Fühler vorspannende Federelement. Gemäß einem anderen Ausführungsbeispiel weist der Leitungskanal für ein Behandlungsfluid eine Bohrung in dem Fühler des Bohrwerkzeugs auf. Bevorzugt weist der Fühler einen Führungsabschnitt, dessen Durchmesser in etwa der lichten Weite der hohlen Außenhülse, in welcher er aufgenommen ist, entspricht, so dass der Führungsabschnitt an der Innenwand der hohlen Außenhülse gelagert ist, und einen zweiten Abschnitt auf, der durch einen Spalt, insbesondere einen Ringspalt, von der Innenwand der hohlen Außenhülse beabstandet ist, wobei der Spalt zumindest einen Teil des Leitungskanals für ein Behandlungsfluid bildet. Besonders bevorzugt sind die Bohrung in dem Fühler, die den Leitungskanal für ein Behandlungsfluid bildet, und der Spalt durch eine Querbohrung in dem Fühler miteinander verbunden.

Diese Ausführungsbeispiele haben den Vorteil, dass trotz der Integration zumindest eines Teils des Sensors in das Bohrwerkzeug eine Leitung des Behandlungsfluids durch das Innere des Bohrwerkzeugs und eine Abgabe des Behandlungsfluids direkt an der Spitze oder am abrasiven Arbeitsende des Bohrwerkzeugs und damit eine besonders effektive Kühlung des Bohrwerkzeugs und der Behandlungsstelle unmittelbar dort, wo das abrasive Arbeitsende des Bohrwerkzeugs am Knochen angreift, realisierbar ist.

Gemäß einem Ausführungsbeispiel weist der Fühler an seinem zweiten, dem Arbeitsende zugewandten Ende eine exzentrisch zur Längsachse angeordnete Fühlerspitze auf. Dies ist insbesondere von Vorteil wenn das Bohrwerkzeug schief auf das zu durchbohrende Material aufgesetzt ist oder die Dicke des zu durchbohrenden Materials variiert, da durch die rotierende, exzentrisch angeordnete Fühlerspitze eine größere Fläche des angebohrten Materials abgetastet wird und somit der dünnste Bereich des Materials besser erkannt werden kann. Die Fühlerspitze kann des Weiteren mit einer Schneide oder einer abrasiven Oberfläche zum Abtragen von Gewebe versehen sein.

Die exzentrisch zur Längsachse angeordnete Fühlerspitze ermöglicht auch eine Drehmitnahme der Fühlerspitze durch die hohle Außenhülse des Bohrwerkzeugs, wobei bevorzugt die exzentrische Fühlerspitze eine zur Längsachse gewandte Mitnehmerfläche aufweist, die eine, insbesondere im Wesentlichen zentrische, Mitnehmerfläche der hohlen Außenhülse kontaktiert, so dass die Antriebsbewegung der hohlen Außenhülse auf den Fühler übertragbar ist und der Fühler sich gemeinsam mit der hohlen Außenhülse dreht.

Eine medizinische, insbesondere dentale, Behandlungsvorrichtung umfasst gemäß einem Ausführungsbeispiel eine Vorrichtung für den Schnellstopp eines medizinischen, insbesondere dentalen, Bohrwerkzeugs, einen Antrieb für das Bohrwerkzeug, ein Handstück zum Anschluss des Bohrwerkzeugs und eine Steuer- und / oder Regelvorrichtung zum Empfang eines von dem elektromagnetischen Sensor erzeugten Sensorsignals und zum Stoppen des Antriebs, wobei zumindest ein Teil des elektromagnetischen Sensors am Handstück angeordnet ist, wobei insbesondere ein Teil des elektromagnetischen Sensors direkt am Handstück befestigt oder befestigbar ist und damit nicht direkt am Bohrwerkzeug befestigt ist. In anderen Worten besteht der Sensor somit aus zwei Teilen, wovon ein Teil fest oder lösbar direkt mit dem Handstück verbunden ist und ein zweiter Teil direkt mit dem Bohrwerkzeug verbunden ist, so dass, wenn das Handstück und das Bohrwerkzeug voneinander getrennt werden, jeweils ein Teil des Sensors am Handstück und ein Teil des Sensors am Bohrwerkzeug verbleibt.

Gemäß einem Ausführungsbeispiel ist der Sensor im Bereich der Werkzeug-Einstecköffnung des Handstücks vorgesehen. Dies ist insbesondere von Vorteil, wenn das Bohrwerkzeug einen Abschnitt mit einem größeren Durchmesser (im Vergleich zum Durchmesser eines anderen Abschnitts des Bohrwerkzeugs) aufweist, zum Beispiel wenn jener Abschnitt einen größeren Durchmesser hat, in oder an dem zumindest ein Teil des Sensors vorgesehen ist. Insbesondere kann die Werkzeug-Einstecköffnung einen Abschnitt mit einem vergrößerten Durchmesser aufweisen, an dem der direkt mit dem Handstück verbundene Teil des Sensors vorgesehen ist und in den der Abschnitt des Bohrwerkzeugs mit dem größeren Durchmesser aufnehmbar ist. Auf diese Weise ist der Sensor am Handstück angeordnet, ohne dass andere Bauteile des Handstücks oder deren Anordnung im Handstück beeinflusst oder geändert werden müssten oder dass andere Bauteile des Handstücks den Sensor oder die Funktion oder den Betrieb des Sensors beeinflussen.

Gemäß einem bevorzugten Ausführungsbeispiel ist ein erster Teil des Sensors am Bohrwerkzeug vorgesehen und durch den Anschluss des Bohrwerkzeugs an das Handstück lösbar mit dem Handstück verbindbar und ist ein zweiter Teil des Sensors über eine lösbare Verbindungsvorrichtung direkt am Handstück befestigbar, insbesondere an der Außenhülse des Handstücks, so dass, wenn der erste und der zweite Teil des Sensors am Handstück befestigt sind, der erste und der zweite Teil des Sensors operativ miteinander in Wechselwirkung treten. Damit ist der Sensor in vorteilhafter Weise vollständig von dem Handstück lösbar und zum Beispiel an unterschiedliche Handstücke anschließbar oder kann separat von dem Handstück gereinigt oder gepflegt werden. Besonders bevorzugt sind der erste Teil des Sensors und / oder der zweite Teil des Sensors an der Außenseite der Außenhülse und / oder außerhalb der Außenhülse des Handstücks anordenbar. Damit kann ein beliebiges Handstück mit dem Sensor oder der Vorrichtung für den Schnellstopp verbunden werden und mit einem beliebigen Handstück ein Bohrer-Schnellstopp durchgeführt werden, ohne dass das Handstück adaptiert werden muss.

Die Erfindung wird nachfolgend anhand bevorzugter Ausführungsbeispiele und Bezug nehmend auf die beigefügten Zeichnungen erläutert:
Figur 1 zeigt eine perspektivische Darstellung eines Ausführungsbeispiels eines medizinischen, insbesondere dentalen, Bohrwerkzeugs einer Vorrichtung für den Schnellstopp.
Die Figuren 2A und 2B zeigen Schnittdarstellungen durch ein Ausführungsbeispiel einer Vorrichtung für den Schnellstopp eines medizinischen, insbesondere dentalen, Bohrwerkzeugs mit einem induktiven Sensor, wobei die Schnittebenen der beiden Figuren 90° zueinander gedreht sind.
Die Figur 3A zeigt eine Außenansicht eines Ausführungsbeispiels einer Vorrichtung für den Schnellstopp eines medizinischen, insbesondere dentalen, Bohrwerkzeugs, wobei das Bohrwerkzeug auf eine Oberfläche aufgesetzt ist, so dass die Fühlerspitze des Fühlers entgegen der Kraft des Federelements in die Außenhülse des Bohrwerkzeugs eingeschoben ist.
Die Figuren 3B und 3C zeigen Schnittdarstellungen durch die Vorrichtung für den Schnellstopp der Figur 3A, wobei die Schnittebenen der beiden Figuren 90° zueinander gedreht sind.
Figur 4 zeigt eine Schnittdarstellung durch ein Ausführungsbeispiel einer Vorrichtung für den Schnellstopp eines medizinischen, insbesondere dentalen, Bohrwerkzeugs mit einem kapazitiven Sensor.
Figur 5 zeigt eine Schnittdarstellung durch ein Ausführungsbeispiel einer Vorrichtung für den Schnellstopp eines medizinischen, insbesondere dentalen, Bohrwerkzeugs mit einem Magnetsensor.
Figur 6 zeigt eine teilweise schematische Darstellung einer Ausführungsform einer medizinischen, insbesondere dentalen, Behandlungsvorrichtung, mit einer Vorrichtung für den Schnellstopp eines Bohrwerkzeugs, einem Antrieb für das Bohrwerkzeug, einem Handstück zum Anschluss des Bohrwerkzeugs und einer Steuer- und / oder Regelvorrichtung zum Empfang eines von dem elektromagnetischen Sensor erzeugten Sensorsignals und zum Stoppen des Antriebs.
Die Figuren 7A, 7B zeigen eine medizinische, insbesondere dentale, Behandlungsvorrichtung, mit einer lösbar mit einem Handstück verbundenen Vorrichtung für den Schnellstopp eines medizinischen, insbesondere dentalen, Bohrwerkzeugs.

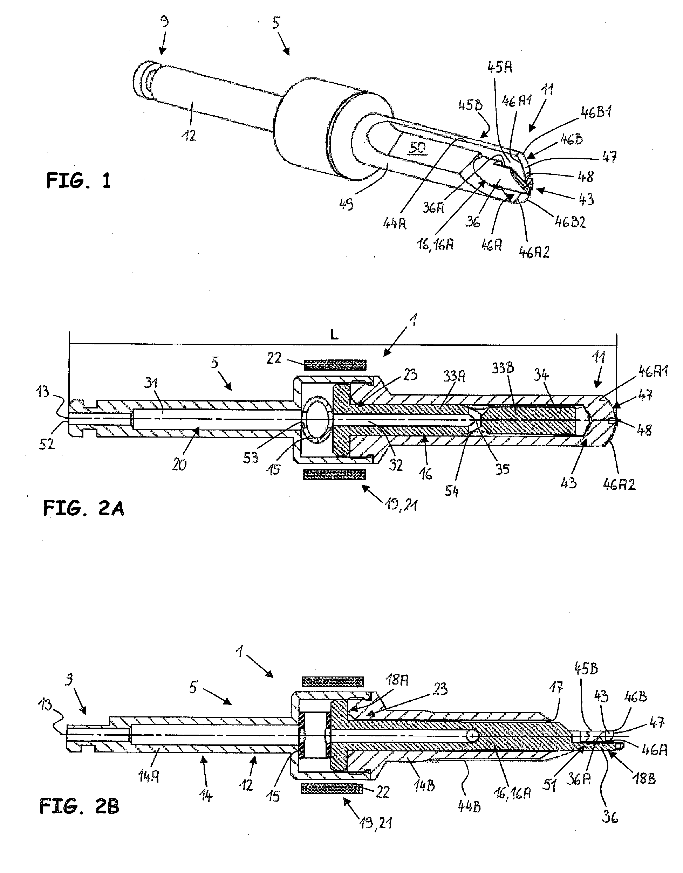

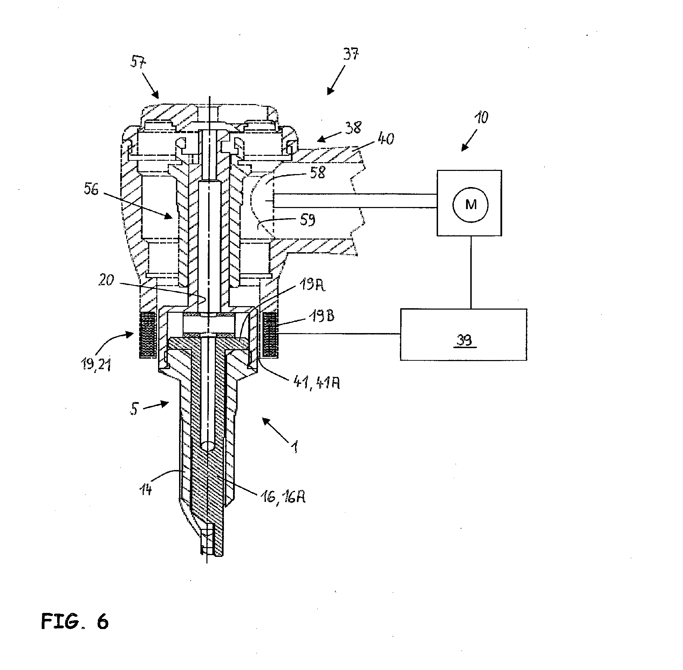

Die Figuren 1, 2A und 2B zeigen einen Bohrer oder ein Bohrwerkzeug 5 wie es für eine Vorrichtung 1, für den Schnellstopp eines medizinischen, insbesondere dentalen, Bohrwerkzeugs 5 verwendbar ist. Das Bohrwerkzeug 5 weist einen länglichen Körper 12 mit einer Längsausdehnung L auf, der sich entlang einer Längsachse oder Mittelachse oder Rotationsachse 13 erstreckt. Die Enden des Körpers 12 sind als Anschlussende 9 zur Verbindung mit einem Antrieb 10 (siehe Figur 6) und als abrasives Arbeitsende 11 zum Materialabtrag ausgebildet. Der Körper 12 besteht des Weiteren aus einer hohlen Außenhülse 14 (siehe Figur 2B) und einem darin aufgenommenen Fühler 16, der relativ zur Außenhülse 14 entlang der Längsachse 13 bewegbar ist.

Der Fühler 16 ist als länglicher, sich entlang der Längsachse 13 erstreckender Stift 16A mit einem ersten, dem Anschlussende 9 zugewandten Ende 18A und einem zweiten, dem Arbeitsende 11 zugewandten Ende 18B ausgebildet. Ein Teil des Fühlers 16 ist durch eine Öffnung 17 (siehe Figur 2B) der Außenhülse 14 am Arbeitsende 11 aus der Außenhülse 14 bewegbar, jedoch ist in verbautem Zustand das erste, dem Anschlussende 9 zugewandte Ende 18A des Fühlers 16 immer im Inneren der hohlen Außenhülse 14 des Bohrwerkzeugs 5 aufgenommen.

Im Folgenden wird das abrasive Arbeitsende 11 des Bohrwerkzeugs 5 beschrieben, das auch einen eigenen, separaten erfinderischen Aspekt darstellt. Ein derartiges abrasives Arbeitsende 11 kann somit auch bei anderen Bohrern oder Bohrwerkzeugen verwendet werden, welche insbesondere keine oder andere als die unten noch im Detail zu beschreibenden Sensoren zur Detektion einer Relativbewegung zwischen der Außenhülse und dem Fühler haben.

Am abrasiven Arbeitsende 11 des Bohrwerkzeugs 5 ist ein Steg 43 vorgesehen, der integraler Teil der hohlen Außenhülse 14 oder mit dieser verbunden ist. Der Steg 43 überspannt die Öffnung 17 der Außenhülse 14 oder die an die Öffnung 17 anschließende im Wesentlichen zylindrische Innenbohrung der Außenhülse 14. Alternativ erstreckt sich der Steg 43 von einer Seite der Außenhülse 14 über die Öffnung 17 oder die Innenbohrung der Außenhülse 14 zu einer zweiten Seite der Außenhülse 14. Bevorzugt ist der Steg 43 mittig an der Außenhülse 14 angeordnet, so dass die Längsachse 13 des Bohrwerkzeugs 5 durch den Steg 43 hindurchtritt (siehe insbesondere Figur 2A). Bevorzugt weist der Steg 43 zwei im Wesentlichen flächig oder plan ausgebildete Seitenwände oder Seitenflächen 45A, 45B auf. Jede Seitenfläche 45A, 45B endet an ihrem distalen, dem Anschlussende 9 abgewandten Ende in einer Kante 46A, 46B (siehe Figur 1), an die eine freie Endfläche 47 des Stegs 43 anschließt. Wie insbesondere aus der Figur 2A zu erkennen ist, ist die freie Endfläche 47 im Querschnitt bevorzugt gebogen oder kreisbogenförmig ausgebildet und weist besonders bevorzugt an ihrem Apex einen Einstich oder Rücksprung 48 mit Kanten auf.

An dem Steg 43 sind eine oder mehrere abrasive Elemente vorgesehen, insbesondere Schneiden. Gemäß einem bevorzugten Ausführungsbeispiel ist jede der beiden Kanten 46A, 46B, insbesondere durch den Einstich 48, in zwei Teile 46A1, 46A2, 46B1, 46B2 unterteilt, wobei jeweils nur eine Teilkante 46A1, 46A2 an der Seitenfläche 45A und eine Teilkante 46B1, 46B2 an der Seitenfläche 45B als abrasive Schneide oder Schneidkante ausgebildet ist. Besonders bevorzugt liegen die beiden als Schneidkanten ausgebildeten Teilkanten (bezogen auf die Mittelachse 13) einander diametral gegenüber, so wie dies beispielhaft in der Figur 1 dargestellt ist: Als Schneidkanten sind die Teilkante 46A1 an der Seitenfläche 45A und die Teilkante 46B2 an der gegenüber liegenden Seitenfläche 45B ausgebildet. Insbesondere sind als Schneiden die jeweils an die Schneidkanten 46A1, 46B2 anschließenden Kanten des Einstichs 48 sowie bevorzugt auch zumindest Teile der an die Schneidkanten 46A1, 46B2 anschließenden und die Seitenflächen 45A, 45B lateral begrenzenden Seitenkanten 44A (siehe Figur 1) und 44B (siehe Figur 2B) ausgebildet. Gemäß einem Ausführungsbeispiel ist jeweils ein unmittelbar an die Schneidkanten 46A1, 46B2 angrenzender Abschnitt der Seitenkanten 44A, 44B mit einer Länge von etwa 0,5 mm - 3,0 mm, bevorzugt von etwa 1,0 mm, als Schneide ausgebildet. Die Teilkanten 46A2, 46B1 sind nicht als Schneidkanten ausgebildet und bevorzugt etwas abgerundet und / oder axial (bezogen auf die Längsachse 13) und relativ zu den Schneidkanten 46A1, 46B2 etwas zurück versetzt.

Zur Ausbildung der abrasiven Seitenkanten 44A, 44B sind an dem zylindrischen Außenmantel 49 des Bohrwerkzeugs 5 zwei flächige Rücksprünge 50 (nur einer davon ist in der Figur 1 zu erkennen) vorgesehen, die bei Betrieb des Bohrwerkzeugs 5 auch zur Abfuhr von durch die Schneidkanten abgelösten Materialspänen, insbesondere Knochenspänen, dienen.

Der Steg 43 ist in den Figuren 1, 2A, 2B als gerades, "I"-förmiges Element mit zwei Kanten 46A, 46 B dargestellt. Selbstverständlich kann der Steg jedoch auch andere Formen aufweisen und / oder mehr Kanten aufweisen, um eine höhere Abrasivität zu erreichen. Zum Beispiel kann der Steg 43 "Y"-förmig ausgebildet sein und somit zumindest drei Schneidkanten aufweisen (an jedem der drei Arme des "Y" eine Schneidkante) oder der Steg 43 kann "X"-förmig ausgebildet sein und somit zumindest vier Schneidkanten aufweisen (an jedem der vier Arme des "X" eine Schneidkante). Gemäß den Figuren 1, 2A, 2B sind die beiden Schneidkanten 46A1, 46B2 um die Breite des Stegs 43 versetzt zueinander angeordnet. Alternativ ist es auch möglich den Steg derart auszuführen, dass die beiden Schneidkanten 46A1, 46B2 eine durchgehende Gerade bilden.

Gemäß dem in den Figuren 1, 2A und 2B dargestellten Ausführungsbeispiel dreht sich der Fühler 16 mit der Außenhülse 14 mit, wenn diese in Drehung versetzt wird, wozu eine Drehmitnahmevorrichtung 51 an dem Bohrwerkzeug 5 vorgesehen ist. Gemäß einem bevorzugten Ausführungsbeispiel sind die Seitenflächen 45A, 45B des Stegs 43 Teil der Drehmitnahmevorrichtung 51, welche die vom Antrieb 10 erzeugte und über das Anschlussende 9 auf die hohlen Außenhülse 14 übertragenen Drehbewegung an den in der Außenhülse 14 aufgenommenen Fühler 16 weiterleiten. Der Fühler 16 weist an seinem zweiten Ende 18B eine Fühlerspitze 36 auf, die exzentrisch zur Längsachse 13 angeordnet ist. An der Fühlerspitze 36 ist zumindest eine im Wesentlichen flächige oder plane Kontaktfläche 36A vorgesehen, die kontaktierend an einer der beiden Seitenflächen 45A, 45B des im Wesentlichen zentral angeordneten Stegs 43 anliegt. Über diesen Kontakt zwischen den Flächen 36A und 45A oder 45B wird die Drehbewegung von der Außenhülse 14 auf den Fühler 16 übertragen. Selbstverständlich können auch andere Arten von Drehmitnahmevorrichtungen 51 vorgesehen sein, zum Beispiel in Form eines Polygons, insbesondere eines Hexagons, wobei ein Abschnitt des Fühlers 16 eine polygone Außenform aufweist und ein korrespondierender Abschnitt der Innenwand der hohlen Außenhülse 14 ebenfalls eine polygone Form hat.

Gemäß dem in den Figuren 1, 2A und 2B dargestellten Ausführungsbeispiel weist der Fühler 16 kein abrasives Element für den Materialabtrag auf und löst somit kein Material von dem zu bearbeitenden Gegenstand, insbesondere Gewebe. Demgemäß ist die Fühlerspitze 36 abgerundet. Selbstverständlich wäre es jedoch gemäß einem anderen Ausführungsbeispiel auch möglich, den Fühler mit einem abrasiven Element für den Materialabtrag zu versehen, insbesondere mit einer Schneidspitze am distalen, dem Material 55 zugewandten Ende.

Um den Fühler 16 und gegebenenfalls weitere Bauteil im Inneren der hohlen Außenhülse 14 des Bohrwerkzeugs 5 anordnen zu können, ist die Außenhülse 14 zweiteilig aufgebaut, wobei die beiden Teile 14A, 14B über geeignete Verbindungsvorrichtungen miteinander verbindbar sind, insbesondere lösbar verbindbar sind. Die Verbindungsvorrichtungen sind zum Beispiel als Schraub-, Steck- oder Bajonettverbindungen ausgebildet. Die beiden Außenhülsenteile 14A, 14B können aus unterschiedlichen oder gleichen Materialien hergestellt sein, zum Beispiel aus Metall, insbesondere Stahl, und / oder Kunststoff, wobei insbesondere jener Außenhülsenteil 14B mit dem abrasiven Ende 11 bevorzugt aus Metall hergestellt ist und gegebenenfalls jener Außenhülsenteil 14A, der den im Weiteren noch zu beschreibenden elektromagnetischen Sensor 19 umgibt, zumindest teilweise aus Kunststoff besteht, um die Funktion des Sensors 19 nicht oder weniger zu beeinträchtigen. Insbesondere wenn der elektromagnetische Sensor 19 als induktiver Sensor 21 ausgebildet ist, ist es von Vorteil zumindest jenen Teil der Außenhülse 14, der im Bereich des induktiven Sensors 21 vorgesehen ist, aus einem nicht-magnetischen Material herzustellen, zum Beispiel aus Aluminium, aus nichtmagnetischem Stahl, aus Kunststoff oder aus Keramik, zum Beispiel aus keramischen Material mit Zirkonium.

In den Figuren 2A, 2B ist eine Vorrichtung 1 für den Schnellstopp eines medizinischen, insbesondere dentalen, Bohrwerkzeugs, insbesondere des in der Figur 1 dargestellten Bohrwerkzeugs 5, dargestellt. Die Vorrichtung 1 umfasst neben dem Bohrwerkzeug 5 auch noch einen elektromagnetischen Sensor 19, der zur Detektion einer Relativbewegung zwischen der Außenhülse 14 und dem Fühler 16 ausgebildet ist. Der Sensor 19 ist entlang oder innerhalb der Längsausdehnung L des Körpers 12 des Bohrwerkzeugs 5 angeordnet. Der Sensor 19 überragt somit die beiden Enden 9, 11 des Bohrwerkzeugs 5 nicht oder nicht wesentlich. Es ist des Weiteren zu erkennen, dass ein Teil des Sensors 19 in dem Bohrwerkzeug 5 und ein weiterer Teil des Sensors 19 außerhalb des Bohrwerkzeugs 5 angeordnet ist.

Gemäß den Figuren 2A, 2B ist der elektromagnetische Sensor 19 als berührungsloser, induktiver Sensor 21 ausgebildet, der zumindest eine Spule 22, die außerhalb des Bohrwerkzeugs 5, jedoch nahe dessen Außenmantel 49 angeordnet ist, und einen Spulenkern 23 umfasst. Die zumindest eine Spule 22 kann das Bohrwerkzeug 5 zum Beispiel ringförmig umgeben. Der Spulenkern 23 umfasst ein Magnetelement, das relativ zur Spule 22 bewegbar ist. Das Magnetelement ist entweder ein separates Element, das mit dem Fühler 16 fest verbunden ist und gemeinsam mit dem Fühler 16 bewegbar ist, oder das Magnetelement wird durch den Fühler 16 gebildet, zum Beispiel in dem der Fühler 16 aus einem magnetischen Material hergestellt ist oder bei der Herstellung magnetisiert wurde, so wie dies für die Figuren 2A und 2B zutrifft. Der Spulenkern 23 ist insbesondere durch das erste Ende 18A des Fühlers 16, das im Inneren der hohlen Außenhülse 14 aufgenommen ist, gebildet.

Der Fühler 16 bzw. der Spulenkern 23 sind durch ein Federelement 15 in Richtung des abrasiven Endes 11 des Bohrwerkzeugs 5 vorgespannt, so dass zumindest ein Teil des Fühlers 16 oder der Fühlerspitze 36 durch die Öffnung 17 nach außen ragt oder die hohle Außenhülse 14, insbesondere den Steg 43, überragt. Das Federelement 15 ist in den Figuren 2A, 2B als aus einem elastischen Material, insbesondere aus elastischem Kunststoff, bestehender Schlauchabschnitt dargestellt, es kann jedoch selbstverständlich auch andere Federn umfassen, insbesondere Spiral- oder Blattfedern, zum Beispiel auch aus Metall.

Die Detektion einer Relativbewegung zwischen der Außenhülse 14 und dem Fühler 16 durch den induktiven Sensor 21 läuft wie folgt ab: Wirkt auf den Fühler 16 eine Kraft, die der Federkraft des Federelements 15 entgegengesetzt ist und die groß genug ist, das Federelement 15 zu komprimieren, dann bewegen sich der Fühler 16 bzw. der Spulenkern 23 bzw. das Magnetelement axial entlang der Längsachse 13 in Richtung des Anschlussendes 9 des Bohrwerkzeugs 5. Der Spulenkern 23 dringt damit in die Spule 22 ein oder dringt weiter in die Spule 22 ein. Die Spule 22 ist mit einer Wechselspannungsquelle verbunden und wird von dieser mit einer Wechselspannung versorgt. Mit dem (weiteren) Eindringen des Spulenkerns 23 oder des Magnetelements in die Spule 22 ändert sich somit deren Induktivität, woraus eine Relativbewegung zwischen der Außenhülse 14 des Bohrwerkzeugs 5 und dem Spulenkern 23 oder dem Fühler 16 abgeleitet werden kann. Die Induktivitätsänderung wird in Form eines Sensorsignals an eine mit dem Sensor 19 verbundene Steuer- und / oder Regelvorrichtung 39 (siehe Figur 6) geleitet, welche auf Basis des Sensorsignals den Antrieb 10 für das Bohrwerkzeug 5 stoppt.

Eine Relativbewegung zwischen der Außenhülse 14 und dem Fühler 16 tritt insbesondere dann auf, wenn das Bohrwerkzeug 5 an einen Übergangsbereich zwischen zwei Materialien mit unterschiedlichen Härten gelangt, insbesondere im medizinischen Bereich an einen Übergangsbereich von einem harten Gewebe, zum Beispiel Knochen, und einem weichen Gewebe, zum Beispiel eine Membran, Muskel- oder Bindegewebe, Gehirnmasse oder gar in einen (gegebenenfalls mit einem Flüssigkeit oder einem Gas gefüllten) Hohlraum. Entsprechende Anwendungsbeispiele, auch für die dentale Anwendung, insbesondere beim Durchbohren des Oberkieferknochens im Rahmen einer Sinusbodenelevation, sind in der eingangs genannten Patentanmeldung DE 10 2008 032 704 A1 beschrieben, so dass diesbezüglich auf diese Schrift verwiesen wird.

Das erste Ende 18A des Fühlers 16 ist bevorzugt radial (bezogen auf die Längsachse 13) verbreitert oder als Flansch oder flanschartige Verbreiterung ausgebildet, um damit eine stabile und ausreichend große Kontaktfläche für das Federelement 15 zu schaffen und / oder eine verbesserte Funktion des Sensors 19 zu bewirken, in dem die innerhalb und außerhalb des Bohrwerkzeugs 5 angeordneten Teile des Sensors 19 räumlich möglichst nahe zueinander zu positionieren. Zumindest das radial verbreiterte Ende 18A, bevorzugt auch das Federelement 15, sind in einem ebenfalls radial verbreiterten Abschnitt des Körpers 12 oder der Außenhülse 14 des Bohrwerkzeugs 5 aufgenommen.

Das Bohrwerkzeug 5 weist einen sich entlang der Längsachse 13 erstreckenden Leitungskanal 20 für ein Behandlungsfluid und / oder Kühlfluid auf. Der Leitungskanal 20 beginnt an einer Öffnung 52 am äußersten Ende des Anschlussendes 9. Über diese Öffnung 52 ist der Leitungskanal 20 mit einer Fluidquelle verbindbar, die zum Beispiel eine physiologische Kochsalzlösung oder ein Anästhetikum oder eine Flüssigkeit zum Anheben einer Membran zur Verfügung stellt. Die Verbindung zur Fluidquelle wird zum Beispiel über ein Röhrchen bewerkstelligt, das durch die Öffnung 52 in den Leitungskanal 20 einführbar ist und das, gegebenenfalls über eine weitere Leitung, mit der Fluidquelle verbunden ist. Gegebenenfalls sind an dem Röhrchen, oder an dem Bohrwerkzeug 5, zum Beispiel an der Öffnung 52 oder im Leitungskanal 20, ein oder mehrere Dichtelemente, zum Beispiel O-Ringe, vorgesehen, die ein Austreten des Fluids aus dem Röhrchen oder dem Leitungskanal 20 verhindern.

Der Leitungskanal 20 verläuft entlang der Längsachse 13 im Inneren der Außenhülse 14 und wird im an die Öffnung 52 anschließenden Abschnitt durch die Innenbohrung 31 der hohlen Außenhülse 14 gebildet. Daran anschließend durchtritt der Leitungskanal 20 das Federelement 15 oder setzt sich in diesem fort oder führt daran vorbei. In dem Ausführungsbeispiel gemäß den Figuren 2A, 2B weist das Federelement 15 eine Durchbohrung 53 auf, so dass das Fluid durch das Federelement 15 und anschließend weiter in eine Bohrung 32 des Fühlers 16 fließen kann. Die Bohrung 32 erstreckt sich ebenfalls axial durch den Fühler 16 entlang der Längsachse 13 und mündet an ihrem der Fühlerspitze 36 zugewandten Ende in eine Querbohrung 35. Die Querbohrung 35 durchsetzt den Fühler 16 radial zur Längsachse 13 und weist zumindest eine Öffnung 54 auf, die in einen Spalt 34 mündet, insbesondere in einen Ringspalt, der durch eine Beabstandung der Außenwand des Fühlers 16 von der Innenwand der hohlen Außenhülse 14 entsteht. Der Spalt 34 endet schließlich in der Öffnung 17, durch die das Fluid aus dem Bohrwerkzeug 5, insbesondere aus der Außenhülse 14, austritt. Alternativ oder zusätzlich ist es möglich, die Bohrung 32 bis zum zweiten Ende 18B des Fühlers 16 zu führen, so dass zumindest ein Teil des Fluids über eine Öffnung im Fühler 16 abgegeben wird.

Um den Spalt 34 bilden zu können, weist der Fühler 16 zwei axial hintereinander angeordnete Abschnitte auf: Einen Führungsabschnitt 33A, dessen Durchmesser in etwa der lichten Weite der hohlen Außenhülse 14 entspricht, so dass der Führungsabschnitt 33A an der Innenwand der hohlen Außenhülse 14 gelagert ist, und einen zweiten Abschnitt 33B, dessen Durchmesser geringer ist als die lichte Weite der hohlen Außenhülse 14, so dass der Fühler 16 in diesem Abschnitt 33B von der Innenwand der Außenhülse 14 beabstandet ist, wodurch der Spalt 34 entsteht. Der Führungsabschnitt 33A dient zum Lagern des Fühlers 16 an der Innenwand der hohlen Außenhülse 14 und zum Führen oder Gleiten des Fühlers 16 in der Außenhülse 14.

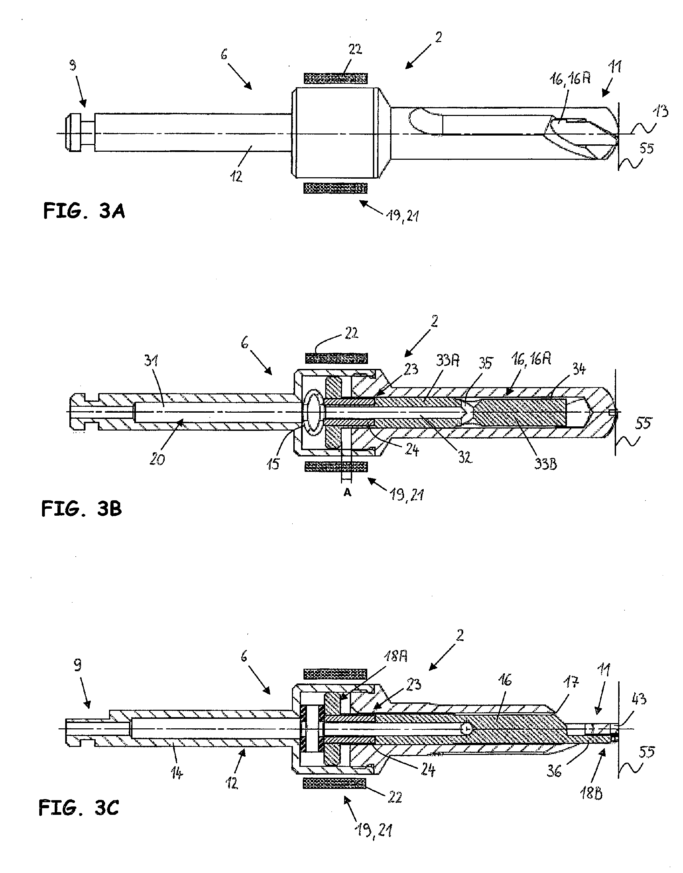

Die Figuren 3A - 3C, 4 und 5 zeigen weitere Vorrichtungen 2, 3, 4 für den Schnellstopp eines medizinischen, insbesondere dentalen, Bohrwerkzeugs 6, 7, 8. Sowohl die Vorrichtungen 2, 3, 4 als auch die Bohrwerkzeuge 6, 7, 8 ähneln in ihrem Aufbau und in ihren Funktionen der im Vorstehenden beschriebenen Schnellstopp-Vorrichtung 1 und dem Bohrwerkzeug 5, so dass gleiche Bauteile mit den gleichen Bezugszeichen versehen sind. Im Folgenden werden daher bevorzugt die abweichenden Merkmale der Schnellstopp-Vorrichtungen 2, 3, 4 beschrieben.

Die in den Figuren 3A - 3C dargestellte Schnellstopp-Vorrichtung 2 umfasst einen elektromagnetischen Sensor 19 zur Detektion einer Relativbewegung zwischen der Außenhülse 14 und dem Fühler 16. Der Sensor 19 ist wiederum als induktiver Sensor 21 ausgebildet, wobei der Spulenkern 23 einen weichmagnetischen Ferritkörper 24 umfasst, welcher bei einer Relativbewegung zwischen dem Fühler 16 und der hohlen Außenhülse 14 bzw. zwischen der Spule 22 und dem Spulenkern 23 die Induktivität der zumindest einen Spule 22 ändert. Der Ferritkörper 24 ist mit dem Fühler 16 verbunden, so dass er sich gemeinsam mit dem Fühler 16 bewegt. Bevorzugt ist der Ferritkörper 24 in einer Ausnehmung des Fühlers 16 aufgenommen. Bevorzugt ist der Ferritkörper 24 als Zylinder oder Hohlzylinder ausgebildet, der konzentrisch am Fühler 16 angeordnet ist. Besonders bevorzugt verläuft der Leitungskanal 20 durch den hohlzylindrischen Ferritkörper 24. Selbstverständlich kann der Ferritkörper jedoch auch andere Formen aufweisen, zum Beispiel als Ferritstab am Fühler 16 befestigt sein.

In den Figuren 3A - 3C ist des Weiteren zu erkennen, dass das Bohrwerkzeug 6 auf ein hartes Material 55 aufgesetzt ist, zum Beispiel ein Knochengewebe. Diese Situation liegt zum Beispiel vor, wenn der Anwender das Bohrwerkzeug 6 vor Beginn des Bohrens auf das Material 55 aufsetzt oder während des Bohrens in dem Material 55. Der Fühler 16 und somit der Spulenkern 23 sind entgegen der Federkraft des Federelements 15 in die hohle Außenhülse 14 (in Richtung des Anschlussendes 9) gedrängt, so dass das freie Ende der Fühlerspitze 36 im Wesentlichen mit dem Steg 43 fluchtet (siehe insbesondere Figur 3C). Das Federelement 15 ist demgemäß komprimiert. Wird das Bohrwerkzeug 6 von dem Material 55 abgehoben oder durchbricht das Bohrwerkzeug 6 das Material 55 oder steht das Bohrwerkzeug 6 kurz davor, das Material 55 zu durchbrechen, dann entspannt sich das Federelement 15 und bewegt den Fühler 16 und den Spulenkern 23 in Richtung des abrasiven Endes 11, so dass zumindest ein Teil der Fühlerspitze 36 die hohle Außenhülse 14, insbesondere den Steg 43, überragt (so wie dies in den Figuren 2A, 2B dargestellt ist). Das Gleiten des Fühlers 16 relativ zur Außenhülse 14 bewirkt die Induktivitätsänderung der Spule 22 und führt zum unverzüglichen Stoppen des Antriebs des Bohrwerkzeugs 5, 6, so wie dies im Vorherstehenden schon beschrieben wurde. Um ein eindeutiges und gut verwertbares Sensorsignal zu erhalten, sollte die Wegänderung A (siehe Figur 3B) des Fühlers 16 oder Spulenkerns 23 zumindest 0,2 mm, bevorzugt zumindest 0,3 mm, betragen.

Gemäß einem Ausführungsbeispiel beträgt der Durchmesser der Spule 22 des induktiven Sensors 21 etwa 5 - 8 mm, die Windungsanzahl der Spule 22 etwa 20 - 40, die Länge des Ferritkörpers 24 etwa 2 - 3 mm und die angelegte Effektivspannung in etwa 0,7 - 1,0 V.

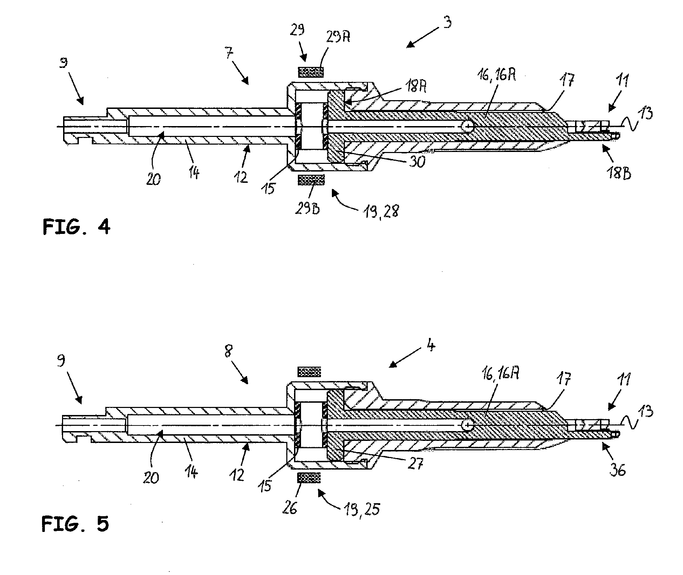

Die in der Figur 4 dargestellte Vorrichtung 3 für den Schnellstopp eines medizinischen, insbesondere dentalen, Bohrwerkzeugs 7 weist einen berührungslosen, elektromagnetischen Sensor 19 zur Detektion einer Relativbewegung zwischen der Außenhülse 14 und dem Fühler 16 auf, der als kapazitiver Sensor 28 ausgebildet ist. Der kapazitive Sensor 28 umfasst zumindest zwei metallischen Elektroden 29, 30, die einen Kondensator bilden, wobei eine Elektrode 30 durch die Relativbewegung des Fühlers 16 zur Außenhülse 14 relativ zu einer anderen Elektrode 29 bewegbar ist. Die Elektroden 29, 30 sind über elektrische Leitungen mit einer Wechselspannungsquelle verbunden und bilden einen hochfrequenten Schwingkreis, an dem ein elektrisches Feld erzeugt wird. Durch die Relativbewegung der beiden Elektroden 29, 30 kommt es zu einer Kapazitätsänderung und damit im Schwingkreis zu einer Veränderung der Verstärkung. Die Kapazitätsänderung bzw. die Veränderung der Verstärkung wird in Form eines Sensorsignals an eine mit dem Sensor 28 verbundene Steuer- und / oder Regelvorrichtung 39 (siehe Figur 6) geleitet, welche auf Basis des Sensorsignals den Antrieb 10 für das Bohrwerkzeug 7 stoppt.

Bevorzugt ist eine Elektrode 30 des Kondensators mit dem Fühler 16 verbunden oder durch den Fühler 16, insbesondere durch einen metallischen Abschnitt des Fühlers 16, gebildet. Die Elektrode 30 ist somit im Bohrwerkzeug 7 aufgenommen. Besonders bevorzugt ist die Elektrode 30 an dem radial verbreiterten Ende 18A des Fühlers 16 vorgesehen oder wird durch dieses Ende 18A gebildet. Eine andere Elektrode 29 ist außerhalb des Bohrwerkzeugs 7, entlang oder innerhalb der Längsausdehnung des Körpers 12 des Bohrwerkzeugs 7 angeordnet. Der kapazitive Sensor 28 überragt somit die beiden Enden 9, 11 des Bohrwerkzeugs 7 nicht oder nicht wesentlich.

Gemäß einem besonders bevorzugten Ausführungsbeispiel weist der kapazitive Sensor 28 zumindest zwei im Wesentlichen plattenförmige Elektroden 29A, 29B sowie eine mit dem Fühler 16 entlang der Längsachse 13 des Bohrwerkzeugs 7 und relativ zu den zwei im Wesentlichen plattenförmigen Elektroden 29A, 29B bewegbare Messelektrode 30 auf. Die plattenförmigen Elektroden 29A, 29B sind außerhalb des Bohrwerkzeugs 7, entlang oder innerhalb der Längsausdehnung des Körpers 12 des Bohrwerkzeugs 7 angeordnet. Die beiden plattenförmige Elektroden 29A, 29B sind insbesondere als Brückenschaltung ausgebildet.

Die in der Figur 5 gezeigte Vorrichtung 4 für den Schnellstopp eines medizinischen, insbesondere dentalen, Bohrwerkzeugs 8 weist einen elektromagnetischen Sensor 19 zur Detektion einer Relativbewegung zwischen der Außenhülse 14 und dem Fühler 16 auf, der als Magnetsensor 25 ausgebildet ist. Der Fühler 16 weist ein Magnetelement 27 auf, das, wie für die Figuren 2A, 2B beschrieben, entweder fest mit dem Fühler 16 verbunden ist, um sich gemeinsam mit dem Fühler 16 zu bewegen, oder das durch den Fühler 16 gebildet ist, zum Beispiel in dem der Fühler 16 aus einem magnetischen Material hergestellt ist oder bei der Herstellung magnetisiert wurde, so wie dies für die Figur 5 zutrifft. Das Magnetelement 27 ist somit wiederum im Inneren der hohlen Außenhülse 14 aufgenommen.

Außerhalb des Bohrwerkzeugs 8, jedoch entlang oder innerhalb der Längsausdehnung des Körpers 12 des Bohrwerkzeugs 7 ist zumindest ein Magnetsensor 26 angeordnet, zum Beispiel ein Hall-Sensor oder ein Reed-Sensor, der einen magnetischen Parameter des Magnetelements 27 detektiert, zum Beispiel die Magnetfeldstärke. Durch die Relativbewegung zwischen der Außenhülse 14 und dem Fühler 16 kommt es zu einer Abstandsänderung zwischen dem Magnetelement 27 und dem Magnetsensor 26 und somit zu einer Änderung des Wertes des magnetischen Parameters an dem Magnetsensor 26. Zum Beispiel nimmt die Magnetfeldstärke des vom Magnetelement 27 erzeugten Magnetfelds am Magnetsensor 26 ab, wenn der Fühler 16 mit dem Magnetelement 27 beim Durchbrechen des gebohrten Materials in Richtung des abrasiven Endes 11 gleitet. Die Veränderung des Wertes des magnetischen Parameters wird wiederum in Form eines Sensorsignals an eine mit dem Sensor 25 verbundene Steuer- und / oder Regelvorrichtung 39 (siehe Figur 6) geleitet, welche auf Basis des Sensorsignals den Antrieb 10 für das Bohrwerkzeug 8 stoppt.

Um die Funktion des Sensors 25 zu unterstützen, ist bevorzugt jener Teil der hohlen Außenhülse 14, an dem der Sensor 25 angeordnet ist, aus nicht metallischem Material hergestellt, zum Beispiel aus Kunststoff.

In der Figur 6 ist eine medizinische, insbesondere dentale, Behandlungsvorrichtung 37 dargestellt, die beispielhaft die Vorrichtung 1 für den Schnellstopp eines medizinischen, insbesondere dentalen, Bohrwerkzeugs 5 umfasst. Selbstverständlich kann die Behandlungsvorrichtung 37 auch die anderen, im Vorstehenden beschriebenen Schnellstopp-Vorrichtungen 2, 3, 4 und die entsprechenden Bohrwerkzeuge 6, 7, 8 aufweisen, so dass die folgende Beschreibung der Figur 6 rein exemplarisch ist und in entsprechender Weise auf die anderen Schnellstopp-Vorrichtungen 2, 3, 4 und die anderen Bohrwerkzeuge 6, 7, 8 zutrifft.

Die Behandlungsvorrichtung 37 umfasst des Weiteren einen Antrieb 10 für das Bohrwerkzeug 5, ein Handstück 38 zum Anschluss des Bohrwerkzeugs 5 und eine Steuer- und / oder Regelvorrichtung 39 zum Empfang eines von dem elektromagnetischen Sensor 19 erzeugten Sensorsignals und zum Stoppen des Antriebs 10. Bevorzugt kann die Behandlungsvorrichtung 37 Vorrichtungen zum Verarbeiten des Sensorsignals des Sensors 19 aufweisen, zum Beispiel zum Glätten, Filtern oder Verstärken des Signals, wobei diese Vorrichtungen insbesondere in der Steuer- und / oder Regelvorrichtung 39 vorgesehen sind oder als Teil der Steuer- und / oder Regelvorrichtung 39 ausgebildet sind.

Das Handstück 38 ist bevorzugt als gewinkeltes Handstück oder Winkelstück mit einer seitlich angeordneten Werkzeug-Einstecköffnung 41 ausgebildet. Das Handstück 38 weist eine hohle Außen- oder Griffhülse 40 auf, in deren Kopfabschnitt eine in eine Arbeitsbewegung versetzbare Werkzeuganschlussvorrichtung oder Spannzange 56 vorgesehen ist. Die Werkzeuganschlussvorrichtung 56 ist insbesondere in Drehung versetzbar und bevorzugt in Wälzlagern gelagert. Mittels einer Lösevorrichtung 57 kann das Bohrwerkzeug 5 wieder aus der Werkzeuganschlussvorrichtung 56 gelöst werden. Insbesondere ist die Werkzeuganschlussvorrichtung 56 als formschlüssige Werkzeuganschlussvorrichtung ausgebildet, zum Beispiel als Aufnahme für einen 2,35 mm Standardbohrer.

Die Werkzeuganschlussvorrichtung 56 ist operativ mit dem Antrieb 10 verbunden, so dass der Antrieb 10 die Werkzeuganschlussvorrichtung 56 und das Bohrwerkzeug 5 in eine Arbeitsbewegung, insbesondere in Rotation, versetzt. Der Antrieb 10 umfasst zum Beispiel einen ansteuerbaren Motor, insbesondere einen Elektromotor, eine oder mehrere Wellen, ein Getriebe, Kupplungen und / oder Zahnräder. Die unmittelbare Übertragung der Antriebsbewegung vom Antrieb 10 auf die Werkzeuganschlussvorrichtung 56 erfolgt über ein am Antrieb vorgesehenes Zahnrad 58 und ein an der Werkzeuganschlussvorrichtung 56 befestigtes Ritzel 59, das mit dem Zahnrad 58 kämmt.

Die Steuer- und / oder Regelvorrichtung 39 ist über elektrische Leitungen und / oder Signalleitungen mit dem Antrieb 10, insbesondere mit dem Motor, und mit dem Sensor 19 verbunden. Die Leitung für den Sensor 19 kann dabei innerhalb des Handstücks 38, insbesondere in der Außenhülse 40, und / oder außerhalb des Handstücks 38 verlaufen.

Die Steuer- und / oder Regelvorrichtung 39 weist eine Energiequelle auf oder ist mit einer Energiequelle verbunden und versorgt den Sensor 19 mit elektrischer Energie, insbesondere mit der benötigten Wechselspannung. Die Steuer- und / oder Regelvorrichtung 39 steuert und / oder regelt den Betrieb des Motors des Antriebs 10, insbesondere versorgt sie den Motor mit Energie oder bestimmt die Drehzahl und / oder das Drehmoment und stoppt den Motor, wenn der elektromagnetische Sensor 19 eine Relativbewegung zwischen der Außenhülse 14 und dem Fühler 16 detektiert. Bevorzugt umfasst die Steuer- und / oder Regelvorrichtung 39 einen Mikrocontroller oder Microcomputer. An der Steuer- und / oder Regelvorrichtung 39 können des Weiteren Stellelemente zum Auswählen oder Einstellen von Betriebsparametern der Behandlungsvorrichtung 37 durch den Anwender und / oder eine Anzeige zum Anzeigen von Betriebsparametern vorhanden sein.

Da während des Bohrvorgangs geringe Relativbewegung zwischen der Außenhülse 14 und dem Fühler 16 auftreten können, welche bereits ein Sensorsignal generieren, ohne dass das Bohrwerkzeug das Material 55 bereits durchbricht oder kurz davor steht, das Material 55 zu durchbrechen, ist bevorzugt in der Steuer- und / oder Regelvorrichtung 39 ein Schwellenwert festgelegt oder durch den Anwender festlegbar, den das Sensorsignal des Sensors 19 überschreiten muss, damit die Steuer- und / oder Regelvorrichtung 39 den Antrieb 10 stoppt.

Gemäß einem Ausführungsbeispiel ist der Sensor 19 im Bereich der Werkzeug-Einstecköffnung 41 des Handstücks 38 vorgesehen.

Gemäß dem Ausführungsbeispiel der Figur 6 ist zumindest ein Teil 19B des elektromagnetischen Sensors 19, 21, insbesondere direkt, am oder im Handstück 38 angeordnet, insbesondere direkt an oder in der Außenhülse 40 des Handstücks 38 befestigt. Der Teil 19B des Sensors 19 bildet somit einen integralen Teil des Handstücks 38, der mit dem Handstück 38 fest verbundenen ist. Im Gegensatz dazu ist der andere Teil 19A des Sensors 19 fest mit dem Bohrwerkzeug 5 verbunden und bildet einen integralen Teil des Bohrwerkzeugs 5. Wird das Bohrwerkzeug 5 mit dem Handstück 38 verbunden, dann sind die beiden Teile 19A, 19B des Sensors 19 derart angeordnet, dass sie operativ miteinander verbunden sind, um eine Relativbewegung zwischen der Außenhülse 14 und dem Fühler 16 zu detektieren.

Gemäß einem bevorzugten Ausführungsbeispiel weist die Werkzeug-Einstecköffnung 41 einen Abschnitt oder Bereich auf, an dem der mit dem Handstücks 38 integrale Teil 19B des Sensors 19 angeordnet ist, zum Beispiel die Spule 22 des induktiven Sensors 21 oder eine Elektrode 29 (Platte 29A, 29B) des kapazitiven Sensors 28. Besonders bevorzugt weist Werkzeug-Einstecköffnung 41 einen verbreiterten Abschnitt 41A auf, an dem der mit dem Handstücks 38 integrale Teil 19B des Sensors 19 angeordnet ist und der zur Aufnahme des anderen Teils 19A des Sensors 19 dient. Insbesondere ist der verbreiterte Abschnitt 41A derart bemessen, dass der Teil 19A des Sensors 19 oder das erst Ende 18A des Fühlers 16 darin aufnehmbar sind. Alternativ weist das Handstück 38, insbesondere die Außenhülse 40, im Bereich der Werkzeug-Einstecköffnung 41 zumindest einen Fortsatz auf, an dem der mit dem Handstücks 38 integrale Teil 19B des Sensors 19 angeordnet ist.

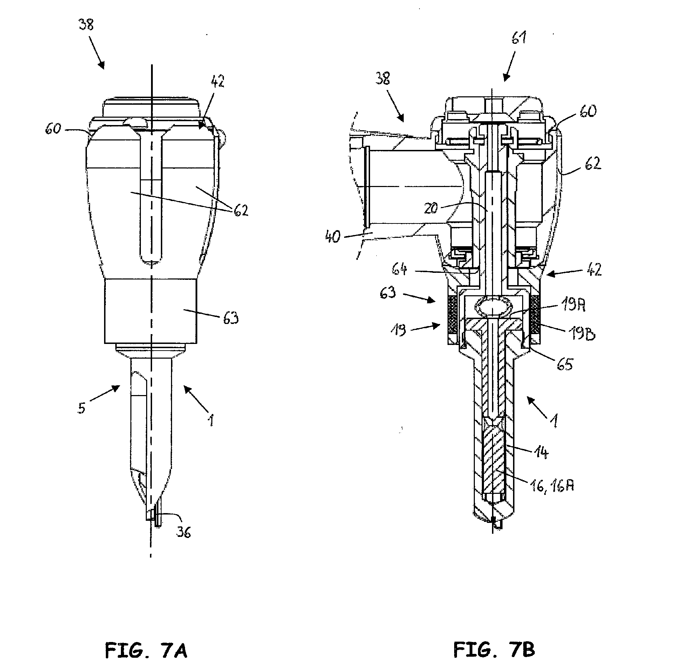

Die Figuren 7A, 7B zeigen ein alternatives Ausführungsbeispiel einer medizinischen, insbesondere dentalen, Behandlungsvorrichtung, bei welcher der gesamte Sensor 19 lösbar mit dem Handstück 38 verbunden ist. Der Sensor 19 ist beispielhaft wieder als induktiver Sensor 21 mit dem Bohrwerkzeug 5 dargestellt, selbstverständlich kann die Behandlungsvorrichtung jedoch auch die anderen, im Vorstehenden beschriebenen Schnellstopp-Vorrichtungen 2, 3, 4 und die entsprechenden Bohrwerkzeuge 6, 7, 8 aufweisen, so dass die folgende Beschreibung der Figuren 7A, 7B wiederum rein exemplarisch ist und in entsprechender Weise auf die anderen Schnellstopp-Vorrichtungen 2, 3, 4 und die anderen Bohrwerkzeuge 6, 7, 8 anwendbar ist. Des Weiteren sind in den Figuren 7A, 7B nur das Handstück 38 und die Schnellstopp-Vorrichtung 1 zu erkennen, jedoch weist auch diese Behandlungsvorrichtung wiederum einen Antrieb, eine Steuer- und / oder Regelvorrichtung und entsprechende elektrische Verbindungen zwischen den Bauteilen auf, wie sie für die Figur 6 beschrieben sind.

Der Sensor 19 weist wiederum zwei Teile 19A, 19B auf, die, wenn sie am Handstück 38 befestigt sind, operativ miteinander verbunden sind, um eine Relativbewegung zwischen der Außenhülse 14 und dem Fühler 16 zu detektieren. Der Teil 19A des Sensors 19 ist fest mit dem Bohrwerkzeug 5 verbunden und bildet einen integralen Teil des Bohrwerkzeugs 5. Durch den Anschluss des Bohrwerkzeugs 5 an das Handstück 38 ist der Teil 19A lösbar mit dem Handstück 38 verbindbar. Der zweite Teil 19B des Sensors 19 ist über eine lösbare Verbindungsvorrichtung 42 direkt am Handstück 38 befestigbar, insbesondere an der Außenhülse 40 des Handstücks 38. Die lösbare Verbindungsvorrichtung 42 kann zum Beispiel als Schraub-, Steck- oder Klemmverbindung ausgebildet sein. Demgemäß sind der erste Teil 19A und der zweite Teil 19B des Sensors 19 an der Außenseite der Außenhülse 40 oder außerhalb der Außenhülse 40 des Handstücks 38 anordenbar.

Gemäß dem in den Figuren 7A, 7B dargestellten bevorzugten Ausführungsbeispiel ist die lösbare Verbindungsvorrichtung 42 als Steckverbindung ausgebildet, die eine Aufnahme 60 zum Einstecken oder Aufnehmen des Kopfabschnitts 61 des Handstücks 38 aufweist. Die Aufnahme 60 umfasst zum Beispiel zwei oder mehrere Federarme 62 zum Befestigen am Kopfabschnitt 61 und einen mit den Federarmen 62 verbundenen Detektionsabschnitt 63 mit dem Teil 19B des Sensors 19. Die Federarme 62 sind insbesondere nach innen, in Richtung der Aufnahme 60 vorgespannt, so dass der Durchmesser der Aufnahme 60 etwas geringer ist als der Durchmesser des Kopfabschnitts 61. Wird der Kopfabschnitt 61 in die Aufnahme 60 gesteckt, so werden die Federarme 62 etwas radial nach außen bewegt und klemmen den Kopfabschnitt 61. Wird der Kopfabschnitt 61 aus der Aufnahme 60 entfernt, so bewegen sich die Federarme 62 wieder radial nach innen in ihre Ausgangsposition.

Der Detektionsabschnitt 63 ist bevorzugt als ringförmiges, hülsenförmiges oder hohlzylindrisches Element ausgebildet, in oder an dem der Teil 19B des Sensors 19 vorgesehen ist, insbesondere die Spule 22 des induktiven Sensors 21 oder eine Elektrode 29 (Platte 29A, 29B) des kapazitiven Sensors 28. Die lösbare Verbindungsvorrichtung 42 ist bevorzugt derart ausgebildet, dass der Detektionsabschnitt 63, insbesondere der Teil 19B des Sensors 19, an der Werkzeug-Einstecköffnung 64 des Handstücks 38 anordenbar ist oder direkt oder bündig daran anschließt. Die lichte Weite der Bohrung 65 des Detektionsabschnitts 63 ist derart bemessen, dass das Bohrwerkzeug 5 darin aufnehmbar ist, insbesondere der am Bohrwerkzeug 5 vorgesehene Teil 19A des Sensors 19 oder das erst Ende 18A des Fühlers 16.

Bei einem bevorzugten Verfahren zum Bohren eines Materials 55 wird eine im Vorstehenden beschriebene Vorrichtung 1, 2, 3, 4 für den Schnellstopp eines medizinischen, insbesondere dentalen, Bohrwerkzeugs 5, 6, 7, 8 oder eine im Vorstehenden beschriebene medizinische, insbesondere dentale, Behandlungsvorrichtung 37 verwendet, wobei der Antrieb des Bohrwerkzeugs 5, 6, 7, 8 gestoppt wird, wenn der elektromagnetischen Sensor 19 eine, insbesondere einen Schwellenwert übersteigende, Relativbewegung zwischen der Außenhülse 14 und dem Fühler 16 des Bohrwerkzeugs 5, 6, 7, 8 detektiert. Bevorzugt ist das Material 55 ein menschliches oder tierisches Gewebe, bevorzugt ein Knochengewebe, insbesondere ein Kieferknochen.

Eine bevorzugte Verwendung der im Vorstehenden beschriebenen Vorrichtung 1, 2, 3, 4 für den Schnellstopp eines medizinischen, insbesondere dentalen, Bohrwerkzeugs 5, 6, 7, 8 oder einer im Vorstehenden beschriebenen medizinischen, insbesondere dentalen, Behandlungsvorrichtung 37 erfolgt beim Durchbohren eines menschlichen oder tierischen Oberkieferknochens, insbesondere im Rahmen einer Sinusbodenelevation.

## Patentansprüche

1. Medizinisches, insbesondere dentales Bohrwerkzeug (5, 6, 7, 8) für eine Vorrichtung (1, 2, 3, 4) für den Schnellstopp des medizinischen, insbesondere dentalen, Bohrwerkzeugs (5, 6, 7, 8), wobei das Bohrwerkzeug (5. 6, 7, 8) umfasst:
Ein Anschlussende (9) zur Verbindung mit einem Antrieb (10), ein abrasives Arbeitsende (11) zum Materialabtrag, einen sich zwischen dem Anschlussende (9) und dem Arbeitsende (11) erstreckenden Körper (12) mit einer sich entlang einer Längsachse (13) des Bohrwerkzeugs (5, 6, 7, 8) erstreckenden Längsausdehnung (L) und eine hohle Außenhülse (14), in welcher ein durch ein Federelement (15) vorgespannter Fühler (16) aufgenommen ist, der relativ zur Außenhülse (14) entlang der Längsachse (13) bewegbar ist, so dass zumindest ein Teil des Fühlers (16) durch eine Öffnung (17) der Außenhülse (14) am Arbeitsende (11) aus der Außenhülse (14) bewegbar ist und wobei die Relativbewegung zwischen der Außenhülse (14) und
dem Fühler (16) durch einen elektromagnetischen Sensor (19) einer Vorrichtung (1, 2, 3, 4) für den Schnellstopp des medizinischen, insbesondere dentalen, Bohrwerkzeugs (5, 6, 7, 8) detektierbar ist, wobei der Fühler (16) als länglicher, sich entlang der Längsachse (13) erstreckender Stift (16A) mit einem ersten, dem Anschlussende (9) zugewandten Ende (18A) und einem zweiten, dem Arbeitsende (11) zugewandten Ende (18B) ausgebildet ist, **dadurch gekennzeichnet, dass** das erste, dem Anschlussende (9) zugewandte Ende (18A) des Fühlers (16) im Inneren der hohlen Außenhülse (14) des Bohrwerkzeugs (5, 6, 7, 8) aufgenommen ist und dass das Bohrwerkzeug (5, 6, 7. 8) einen sich entlang der Längsachse (13) erstreckenden Leitungskanal (20) für ein Behandlungsfluid aufweist.

2. Bohrwerkzeug (5, 6, 7, 8) nach Anspruch 1, **gekennzeichnet durch** einen Spulenkern (23), zum Beispiel ein hart- oder weichmagnetisches Magnetelement, insbesondere einen Ferritkörper (24), welcher mit dem Fühler (16) entlang der Längsachse (13) des Bohrwerkzeugs (5, 6, 7, 8) und relativ zu zumindest einer Spule (22) der Vorrichtung (1, 2, 3, 4) für den Schnellstopp bewegbar ist.

3. Bohrwerkzeug (5, 6, 7, 8) nach Anspruch 1, **gekennzeichnet durch** zumindest ein Magnetelement (27), das **durch** die Relativbewegung des Fühlers (16) zur Außenhülse (14) bewegbar ist.

4. Bohrwerkzeug (5, 6, 7, 8) nach Anspruch 1, **gekennzeichnet durch** eine Elektrode (30), welche **durch** die Relativbewegung des Fühlers (16) zur Außenhülse (14) relativ zu einer anderen Elektrode (29) der Vorrichtung (1, 2, 3, 4) für den Schnellstopp bewegbar ist.

5. Bohrwerkzeug (5, 6, 7, 8) nach einem der vorherstehenden Ansprüche, **dadurch gekennzeichnet, dass**
der Leitungskanal (20) für ein Behandlungsfluid eine Bohrung (31) in der Außenhülse (14) des Bohrwerkzeugs (5, 6, 7, 8) aufweist und / oder das den Fühler (16) vorspannende Federelement (16) durchsetzt.

6. Bohrwerkzeug (5, 6, 7, 8) nach einem der vorstehenden Ansprüche, **dadurch gekennzeichnet, dass**
der Leitungskanal (20) für ein Behandlungsfluid eine Bohrung (32) in dem Fühler (16) des Bohrwerkzeugs (5, 6, 7, 8) aufweist.

7. Bohrwerkzeug (5, 6, 7, 8) nach einem der vorstehenden Ansprüche, **dadurch gekennzeichnet, dass**
der Fühler (16) einen Führungsabschnitt (33A) aufweist, dessen Durchmesser in etwa der lichten Weite der hohlen Außenhülse (14) entspricht, so dass der Führungsabschnitt (33A) an der Innenwand der hohlen Außenhülse (14) gelagert ist, und dass der Fühler (16) einen zweiten Abschnitt (33B) aufweist, der durch einen Spalt (34) insbesondere einen Ringspalt, von der Innenwand der hohlen Außenhülse (14) beabstandet ist, wobei der Spalt (34) zumindest einen Teil des Leitungskanals (20) für ein Behandlungsfluid bildet.

8. Bohrwerkzeug (5, 6, 7, 8) nach den Ansprüchen 6 und 7, **dadurch gekennzeichnet, dass**
die Bohrung (32) in dem Fühler (16), die den Leitungskanal (20) für ein Behandlungsfluid bildet, und der Spalt (34) durch eine Querbohrung (35) in dem Fühler (16) miteinander verbunden sind.

9. Bohrwerkzeug (5, 6, 7, 8) nach einem der vorherstehenden Ansprüche, **dadurch gekennzeichnet, dass**
der Fühler (16) an seinem zweiten, dem Arbeitsende (11) zugewandten Ende (18B) eine exzentrisch zur Längsachse (13) angeordnete Fühlerspitze (36) aufweist.

10. Bohrwerkzeug (5, 6, 7, 8) nach einem der vorherstehenden Ansprüche, **dadurch gekennzeichnet, dass**
am abrasiven Arbeitsende (11) des Bohrwerkzeugs (5) ist ein Steg (43) vorgesehen ist, der integraler Teil der hohlen Außenhülse (14) oder mit dieser verbunden ist, wobei sich der Steg (43) von einer Seite der Außenhülse (14) über die Öffnung (17) oder eine Innenbohrung der Außenhülse (14) zu einer zweiten Seite der Außenhülse (14) erstreckt.

11. Bohrwerkzeug (5, 6, 7, 8) nach Anspruch 10, **dadurch gekennzeichnet dass** an dem Steg (43) eine oder mehrere abrasive Elemente vorgesehen sind, insbesondere Schneiden.

12. Bohrwerkzeug (5, 6, 7, 8) nach einem der vorherstehenden Ansprüche, **gekennzeichnet durch**
eine Drehmitnahmevorrichtung (51) an dem Bohrwerkzeug (5, 6, 7, 8), so dass der Fühler (16) mit der Außenhülse (14) mitdrehbar ist, wenn die Außenhülse (14) in Drehung versetzt wird.

13. Bohrwerkzeug (5, 6, 7, 8) nach Anspruch 12, **dadurch gekennzeichnet, dass** die exzentrische Fühlerspitze (36) eine zur Längsachse (13) gewandte Mitnehmerfläche (36A) aufweist, die eine, insbesondere im Wesentlichen zentrische, Mitnehmerfläche (45A, 45B) der hohlen Außenhülse (14) kontaktiert, so dass die Antriebsbewegung der hohlen Außenhülse (14) auf den Fühler (16) übertragbar ist und der Fühler (16) sich gemeinsam mit der hohlen Außenhülse (14) dreht.

## Claims

1. A medical, in particular dental drilling tool (5, 6, 7, 8) for a device (1, 2, 3, 4) for the quick stop of the medical, in particular dental drilling tool (5, 6, 7, 8), wherein the drilling tool (5, 6, 7, 8) comprises:
a connection end (9) for connecting to a drive (10), an abrasive working end (11) for removing material, a body (12) extending between the connection end (9) and the working end (11) having a longitudinal extension (L) extending along a longitudinal axis (13) of the drilling tool (5, 6, 7, 8) and a hollow outer shell (14) in which a probe (16) pre-tensioned by a spring element (15) is held, which is movable along the longitudinal axis (13) relative to the outer shell (14), so that at least a part of the probe (16) is movable out of the outer shell (14) through an opening (17) in the outer shell (14) at the working end (11), and wherein the relative motion between the outer shell (14) and the probe (16) can be detected by an electromagnetic sensor (19) of a device (1, 2, 3, 4) for the quick stop of the medical, in particular dental drilling tool (5, 6, 7, 8), wherein the probe (16) is designed as an elongated pin (16A) extending along the longitudinal axis (13) having a first end (18A) facing the connection end (9) and a second end (18B) facing the working end (11), **characterized in that** the first end (18A) of the probe (16) facing the connection end (9) is held in the interior of the hollow outer shell (14) of the drilling tool (5, 6, 7, 8) and that the drilling tool (5, 6, 7, 8) comprises a channel (20) for a treatment fluid extending along the longitudinal axis (13).

2. A drilling tool (5, 6, 7, 8) according to claim 1, **characterized by** a coil core (23), for example a hard- or soft-magnetic magnetic element, in particular a ferrite body (24), which can be moved with the probe (16) along the longitudinal axis (13) of the drilling tool (5, 6, 7, 8) and relative to at least one coil (22) of the device (1, 2, 3, 4) for the quick stop.

3. A drilling tool (5, 6, 7, 8) according to claim 1, **characterized by** at least one magnetic element (27) which can be moved through the movement of the probe (16) relative to the outer shell (14).

4. A drilling tool (5, 6, 7, 8) according to claim 1, **characterized by** an electrode (30) which can be moved relative to another electrode (29) of the device (1, 2, 3, 4) for the quick stop through the movement of the probe (16) relative to the outer shell (14).

5. A drilling tool (5, 6, 7, 8) according to one of the preceding claims, **characterized in that**
the channel (20) for a treatment fluid comprises a bore (31) in the outer shell (14) of the drilling tool (5, 6, 7, 8) and/or passes through the spring element (15) which pretensions the probe (16).

6. A drilling tool (5, 6, 7, 8) according to one of the preceding claims, **characterized in that**
the channel (20) for a treatment fluid comprises a borehole (32) in the probe (16) of the drilling tool (5, 6, 7, 8).

7. A drilling tool (5, 6, 7, 8) according to one of the preceding claims, **characterized in that**
the probe (16) comprises a guide section (33A), whose diameter corresponds approximately to the inner width of the hollow outer shell (14), so that the guide section (33A) is supported on the inner wall of the hollow outer shell (14), and that the probe (16) comprises a second section (33B) that is separated from the inner wall of the hollow outer shell (14) by a gap (34), particularly an annular gap, wherein the gap (34) forms at least a part of the channel (20) for a treatment fluid.

8. A drilling tool (5, 6, 7, 8) according to one of claims 6 and 7, **characterized in that** the borehole (32) in the probe (16) which forms the channel (20) for a treatment fluid and the gap (34) are connected with one another through a cross hole (35) in the probe (16).

9. A drilling tool (5, 6, 7, 8) according to one of the preceding claims, **characterized in that**
the probe (16) comprises at its second end (18B) facing the working end (11) a probe tip (36) arranged eccentrically to the longitudinal axis (13).

10. A drilling tool (5, 6, 7, 8) according to one of the preceding claims, **characterized in that**
a bar (43) is provided at the abrasive working end (11) of the drilling tool (5) that is an integral component of the hollow outer shell (14) or is connected with the hollow outer shell (14), wherein the bar (43) extends from one side of the outer shell (14) over the opening (17) or an internal bore of the outer shell (14) to a second side of the outer shell (14).

11. A drilling tool (5, 6, 7, 8) according to claim 10, **characterized in that** one or more abrasive elements are provided on the bar (43), in particular blades.

12. A drilling tool (5, 6, 7, 8) according to one of the preceding claims, **characterized by** a rotating cam mechanism (51) on the drilling tool (5, 6, 7, 8), so that the probe (16) is rotated along with the outer shell (14) when the outer shell (14) is set in motion.

13. A drilling tool (5, 6, 7, 8) according to claim 12, **characterized in that** the eccentric probe tip (36) comprises a cam surface (36A) facing the longitudinal axis (13) which is in contact with a cam surface (45A, 45B) of the hollow outer shell (14), said cam surface (45A, 45B) being in particular centrically, so that the drive movement of the hollow outer shell (14) can be transferred to the probe (16) and the probe (16) rotates along with the outer shell (14).

## Revendications

1. Outil de forage médical, en particulier dentaire (5, 6, 7, 8), pour un dispositif (1, 2, 3, 4) pour l'arrêt rapide de l'outil de forage médical, en particulier dentaire (5, 6, 7, 8), dans lequel l'outil de forage (5, 6, 7, 8) comprend:
une extrémité de raccordement (9) pour la liaison avec un mécanisme de commande (10), une extrémité de travail abrasive (11) pour l'enlèvement de matière, un corps (12) s'étendant entre l'extrémité de raccordement (9) et l'extrémité de travail (11) avec une extension longitudinale (L) s'étendant le long d'un axe longitudinal (13) de l'outil de forage (5, 6, 7, 8), et une enveloppe extérieure creuse (14) dans laquelle est logée une sonde (16) précontrainte par un élément de ressort (15), laquelle sonde peut se déplacer par rapport à l'enveloppe extérieure (14) le long de l'axe longitudinal (13) de manière à ce qu'au moins une partie de la sonde (16) puisse se déplacer hors de l'enveloppe extérieure (14) à travers une ouverture (17) de l'enveloppe extérieure (14) au niveau de l'extrémité de travail (11), et dans lequel il est possible de détecter le mouvement relatif entre l'enveloppe extérieure (14) et la sonde (16) grâce à un capteur électromagnétique (19) d'un dispositif (1, 2, 3, 4) pour l'arrêt rapide de l'outil de forage médical, en particulier dentaire (5, 6, 7, 8), dans lequel la sonde (16) est réalisée en tant que tige (16A) oblongue s'étendant le long de l'axe longitudinal (13) avec une première extrémité (18A) tournée vers l'extrémité de raccordement (9) et une deuxième extrémité (18B) tournée vers l'extrémité de travail (11), **caractérisé en ce que**
la première extrémité (18A) de la sonde (16) tournée vers l'extrémité de raccordement (9) est logée à l'intérieur de l'enveloppe extérieure creuse (14) de l'outil de forage (5, 6, 7, 8) et **en ce que** l'outil de forage (5, 6, 7, 8) présente un canal de conduction (20) s'étendant le long de l'axe longitudinal (13) pour un fluide de traitement.

2. Outil de forage (5, 6, 7, 8) selon la revendication 1, **caractérisé par** un noyau de bobine (23), par exemple un élément d'aimant magnétiquement dur ou doux, en particulier un corps de ferrite (24), lequel peut se déplacer avec la sonde (16) le long de l'axe longitudinal (13) de l'outil de forage (5, 6, 7, 8) et par rapport à au moins une bobine (22) du dispositif (1, 2, 3, 4) pour l'arrêt rapide.

3. Outil de forage (5, 6, 7, 8) selon la revendication 1, **caractérisé par** au moins un élément d'aimant (27) qui peut se déplacer grâce au mouvement relatif de la sonde (16) par rapport à l'enveloppe extérieure (14).

4. Outil de forage (5, 6, 7, 8) selon la revendication 1, **caractérisé par** une électrode (30), laquelle peut se déplacer, grâce au mouvement relatif de la sonde (16) par rapport à l'enveloppe extérieure (14), par rapport à une autre électrode (29) du dispositif (1, 2, 3, 4) pour l'arrêt rapide.

5. Outil de forage (5, 6, 7, 8) selon l'une des revendications précédentes, **caractérisé en ce que**
le canal de conduction (20) pour un fluide de traitement présente une forure (31) dans l'enveloppe extérieure (14) de l'outil de forage (5, 6, 7, 8) et/ou traverse l'élément de ressort (15) précontraignant la sonde (16).

6. Outil de forage (5, 6, 7, 8) selon l'une des revendications précédentes, **caractérisé en ce que**
le canal de conduction (20) pour un fluide de traitement présente une forure (32) dans la sonde (16) de l'outil de forage (5, 6, 7, 8).

7. Outil de forage (5, 6, 7, 8) selon l'une des revendications précédentes, **caractérisé en ce que**
la sonde (16) présente un segment de conduction (33A) dont le diamètre correspond sensiblement au diamètre intérieur de l'enveloppe extérieure creuse (14), de sorte que le segment de conduction (33A) est logé au niveau de la paroi intérieure de l'enveloppe extérieure creuse (14), et **en ce que** la sonde (16) présente un deuxième segment (33B), lequel est espacé par rapport à la paroi intérieure de l'enveloppe extérieure creuse (14) grâce à un interstice (34), en particulier un interstice annulaire, dans lequel l'interstice (34) forme au moins une partie du canal de conduction (20) pour un fluide de traitement.

8. Outil de forage (5, 6, 7, 8) selon les revendications 6 et 7, **caractérisé en ce que** la forure (32) dans la sonde (16), laquelle forme le canal de conduction (20) pour un fluide de traitement, et l'interstice (34) sont reliés ensemble grâce à un forage transversal (35) dans la sonde (16).

9. Outil de forage (5, 6, 7, 8) selon l'une des revendications précédentes, **caractérisé en ce que**
la sonde (16) présente, sur sa deuxième extrémité (18B) tournée vers l'extrémité de travail (11), une pointe de sonde (36) disposée de manière excentrique par rapport à l'axe longitudinal (13).

10. Outil de forage (5, 6, 7, 8) selon l'une des revendications précédentes, **caractérisé en ce qu'**
une arête (43) est prévue au niveau de l'extrémité de travail abrasive (11) de l'outil de forage (5), laquelle fait partie intégrante de l'enveloppe extérieure creuse (14) ou bien est reliée à celle-ci, dans lequel la arête (43) s'étend à partir d'un côté de l'enveloppe extérieure (14), via l'ouverture (17) ou une forure intérieure de l'enveloppe extérieure (14), vers un deuxième côté de l'enveloppe extérieure (14).

11. Outil de forage (5, 6, 7, 8) selon la revendication 10, **caractérisé en ce que** l'on prévoit un ou plusieurs éléments abrasifs au niveau de l'arête (43), en particulier lames.

12. Outil de forage (5, 6, 7, 8) selon l'une des revendications précédentes, **caractérisé par**
un dispositif d'entraînement en rotation (51) au niveau de l'outil de forage (5, 6, 7, 8), de sorte que la sonde (16) peut tourner ensemble avec l'enveloppe extérieure (14) lorsque l'enveloppe extérieure (14) est mise en rotation.

13. Outil de forage (5, 6, 7, 8) selon la revendication 12, **caractérisé en ce que** la pointe de sonde excentrique (36) présente une surface d'entraînement (36A) tournée vers l'axe longitudinal (13), laquelle entre en contact avec une surface d'entraînement (45A, 45B), en particulier sensiblement centrale, de l'enveloppe extérieure creuse (14) de manière à ce que le mouvement d'entraînement de l'enveloppe extérieure creuse (14) puisse être transmis à la sonde (16) et que la sonde (16) tourne en commun avec l'enveloppe extérieure creuse (14).
